Europäisches Patentamt

European Patent Office

Office européen des brevets

(19)

(11) **EP 0 591 281 B1**

(12) **FASCICULE DE BREVET EUROPEEN**

(45) Date de publication et mention
de la délivrance du brevet:
**29.09.1999 Bulletin 1999/39**

(51) Int Cl.⁶: **C07K 14/16**, C07K 14/52,
C07K 14/56, C12N 7/06,
C12P 21/02, C07K 7/06,
C07K 7/08, A61K 39/00,
A61K 39/21

(21) Numéro de dépôt: **92912431.1**

(22) Date de dépôt: **17.06.1992**

(86) Numéro de dépôt international:
**PCT/FR92/00544**

(87) Numéro de publication internationale:
**WO 92/22577 (23.12.1992 Gazette 1992/32)**

(54) **COMPOSES IMMUNOGENES A EFFET NOTAMMENT ANTI-CYTOKINE, PROCEDE DE PREPARATION, COMPOSITIONS PHARMACEUTIQUES ET KITS LES RENFERMANT**

IMMUNOGENE VERBINDUNGEN MIT SPEZIFISCHEM ANTI-CYTOKIN EFFEKT, METHODE ZUR HERSTELLUNG, PHARMAZEUTISCHE ZUSAMMENSETZUNG UND REAKTIONSKIT DER DIESE ENTHÄLT

IMMUNOGEN COMPOUNDS HAVING SPECIFICALLY AN ANTI-CYTOKINE EFFECT, METHOD OF PREPARATION, PHARMACEUTICAL COMPOSITIONS AND KITS CONTAINING THEM

(84) Etats contractants désignés:
**AT BE CH DE DK ES FR GB GR IT LI LU MC NL SE**

(30) Priorité: **17.06.1991 FR 9107399**

(43) Date de publication de la demande:
**13.04.1994 Bulletin 1994/15**

(73) Titulaire: **NEOVACS**
**75116 Paris (FR)**

(72) Inventeurs:
• **ZAGURY, Daniel**
**F-75007 Paris (FR)**
• **ZAGURY, Jean-François**
**F-75003 Paris (FR)**
• **CARELLI, Claude**
**F-92150 Suresnes (FR)**
• **HALBREICH, Abraham**
**F-92800 Puteaux (FR)**
• **BIZZINI, Bernard 19, rue d'Elancourt**
**F-78320 Le Mesnil-Saint-Denis (FR)**

(74) Mandataire: **Santarelli, Marc et al**
**Cabinet Rinuy et Santarelli**
**14, avenue de la Grande Armée**
**75017 Paris (FR)**

(56) Documents cités:
**EP-A- 0 273 716**   **EP-A- 0 323 157**
**EP-A- 0 330 359**   **EP-A- 0 387 914**
**EP-A- 0 398 707**   **EP-A- 0 459 842**
**WO-A-88/00471**     **WO-A-88/05783**
**WO-A-89/12066**     **WO-A-91/05860**
**WO-A-92/00098**     **GB-A- 2 157 697**
**US-A- 4 833 165**

• **NUCLEIC ACIDS RESEARCH. vol. 11, no. 3, Février 1983, ARLINGTON, VIRGINIA US pages 555 - 573 G. D. SHAW ET AL 'Structure and expression of cloned murine IFN-alpha genes'**
• **CHEMICAL ABSTRACTS, vol. 114, no. 3, 21 Janvier 1991, Columbus, Ohio, US; abstract no. 22025m, LOLEIT M. ET AL 'Conjugates of synthetic lymphocyte-activating lipopeptides with segments from HIV proteins induce protein-specific antibody formation' page 527 ;colonne L ;**

Remarques:
Le dossier contient des informations techniques présentées postérieurement au dépôt de la demande et ne figurant pas dans le présent fascicule.

**Description**

Nucleic Acid Reasearch, Vol 11, N° 3, Février 1993, Arlington, Virginia US pages 555-573 décrit des molécules natives d'interféron α1 et α2 murins. GB-A-2 157 697 décrit des molécules natives d'interférons α bovins. WO-A-8 805 783 décrit des peptides homologues à un morceau de la molécule rétrovirale du virus murin MLV p15E, ces peptides étant dotés de propriétés suppressives, de même que l'utilisation de ces peptides comme agents immunisants contre une infection rétrovirale ou au contraire pour induire une immunosuppression.

La présente invention concerne des composés immunogènes à effet notamment anti-cytokine, un procédé de préparation, compositions pharmaceutiques et kits les renfermant.

La présente invention a un double objectif :

1) Des composés préparés et utilisés tout comme des agents d'immunisation active(vaccination). Ce sont :

- des composés dérivés d'une cytokine par perte de l'activité biologique caractéristique de la cytokine, qui sont capables d'induire une réponse immunitaire in vivo à l'encontre de la cytokine native,
- des sites peptidiques du HIV-1 sélectionnés du fait de leur interférence éventuelle avec les processus d'activation des cellules T,
- des particules de virus HIV déplétés de leur ARN génomique, ainsi que des compositions pharmaceutiques les contenant.

2) Un traitement prophylactique ou thérapeutique consistant en une vaccination anti-cytokine et visant à réparer les dérèglements homéostatiques provoqués par la surproduction d'une cytokine. Cette stratégie nouvelle de vaccination pourra être utilisée seule ou, dans le cas d'une infection microbienne, combinée à une vaccination conventionnelle (immunisation active dirigée contre l'agent infectieux).

Les cytokines sont des protéines modulatrices de l'activité ou de la prolifération cellulaire, dont la production est généralement locale et transitoire et qui agissent de manière paracrine ou autocrine. Dans ce qui suit, le terme "cytokine" rassemble des familles de molécules endogènes à dénomination variée : lymphokines, monokines, interleukines, interférons, facteurs de colonisation et facteurs de croissance, neuro peptides.

Les cytokines connues sont notamment l'interféron-α (IFN-α), l'interféron-β (IFN-β), l'interféron-gamma (IFN-gamma), l'interleukine-1 (IL-1) sous types α et β, l'interleukine-2 (IL-2), l'interleukine-3 (IL-3), l'interleukine-4 (IL-4), l'interleukine-5 (IL-5), l'interleukine-6 (IL-6), l'interleukine 10 (IL-10), le tumor necrosis factor (TNF) sous types α et β, le transforming growth factor-β (TGF-β), sous types β1, β2, β3, β1.2, et les colony-stimulating factors (CSF) tels que le granulocyte macrophage-stimulating factor (GM-CSF), le granulocyte colony-stimulating factor (G-CSF) et le macrophage-stimulating factor (M-CSF) et le facteur de croissance épithélial (EGF), la somatostine, les endorphines, les divers "releasing factors" ou "inhibitory factors" tels que le TRF.

Certains états pathologiques dus à une infection, tels que le SIDA ou l'Herpès, peuvent découler de dérèglements homéostatiques induits par une surproduction de cytokine(s) comme l'α-IFN ou le TNF. D'autres états pathologiques (tumeur maligne, allergie, maladie auto-immune) peuvent être également la conséquence de dérèglements homéostatiques.

Afin de traiter certains de ces états pathologiques, l'usage d'anticorps monoclonaux (MAbs) neutralisants anti-cytokines a été proposé à des fins thérapeutiques. Certains d'entre eux ont déjà été expérimentés avec succès. De tels MAbs sont destinés à neutraliser l'hyperproduction d'une cytokine.

A cette thérapie passive de type sérothérapique, on propose maintenant de substituer un thérapie active de type vaccinal qui consiste en l'administration d'une cytokine immunogène, inactivée ou d'un de ses analogues inactifs. Il s'agit en fait d'induire un réponse immunitaire de l'organisme à l'encontre de la cytokine native qui est produite en excès. Cette thérapie active, tout comme l'administration de MAbs, mettrait en oeuvre un effet "tampon," mais certainement de manière plus fine et plus adaptée. En outre, cette nouvelle thérapie évite la nécessité d'humaniser les MAbs d'origine animale (murine) qui est une étape longue et délicate dans le développement des MAbs à fins thérapeutiques.

D'autre part l'IFN-α, -β ainsi que le TGF-β sont en particulier connus comme inhibiteurs de la croissance des cellules de l'immunité (cytostase).

Le système immunitaire se présente sous deux aspects fonctionnels : l'immunité non-spécifique et l'immunité spécifique, dite encore à mémoire. L'immunité non spécifique consiste en une première ligne de défense, capable d'arrêter la plupart des agents pathogènes avant que ne s'établisse une véritable infection. Les mécanismes de l'immunité spécifique entrent ensuite en action. Ils déclenchent une réaction dirigée spécifiquement contre le germe responsable, entraînant sa destruction.

Les phénomènes de l'immunité spécifique et non-spécifique sont le fait de cellules et de molécules réparties dans tout l'organisme. L'immunité non-spécifique met notamment en jeu des cellules phagocytaires et des cellules tueuses ou NK (natural killer) ainsi que des facteurs solubles tels que le lysosyme, le complément et une famille d'agents antiviraux à large spectre d'action c'est à dire les interférons.

Par la suite, on a mis en évidence que les interférons peuvent être produits dans des conditions physiologiques ou physiopathologiques qui ne sont pas forcément associées à des infections virales. Il existe, en effet, un certain nombre d'inducteurs de la synthèse des interférons qui sont des substances non-virales.

Les interférons possèdent outre leur effet antiviral dans le simple contrôle d'une infection, une activité anti-proliférative (cytostatique) in vivo qui leur confère des propriétés anti-tumorale, et immunorégulatrice des réactions immunitaires spécifiques. On s'accorde toutefois à penser que cet effet régulateur est extrêmement complexe.

L'immunité spécifique acquise est quant à elle le fait d'une coopération des macrophages, des lymphocytes B (immunité à médiation humorale) et des lymphocytes T (immunité à médiation cellulaire).

D'une manière générale et schématique, il existe deux grands types de réponse immunitaire spécifique : la réponse de type humoral qui est caractérisée par la production d'anticorps par les lymphocytes B et la réponse immunitaire à médiation cellulaire qui met en jeu des cellules effectrices, c'est à dire essentiellement les lymphocytes $T_8$ (lymphocytes cytotoxiques). Ces réponses sont initialement activées par les cellules présentatrices d'antigènes et modulées par les cellules régulatrices, c'est à dire les lymphocytes $T_4$ (lymphocytes T auxilliaires) et les lymphocytes T suppresseurs.

Dans ses très grandes lignes, la réponse immunitaire spécifique fonctionne comme suit :

- Les cellules présentatrices d'antigène (monocytes, macrophages et lymphocytes B) capturent l'antigène, le digèrent et en réexposent des fragments à leur surface, en association avec des molécules du complexe majeur d'histocompatibilité (CMH) de classe I ou II.

- Quand les lymphocytes $T_4$ "voient" les fragments d'antigènes associés au complexe majeur d'histocompatibilité de classe II, ils prolifèrent, passent sous forme activée (synthétisent de l'IL-2), et ce faisant stimulent la prolifération des lymphocytes B producteurs d'anticorps et celle des lymphocytes $T_8$ (lymphocytes cytotoxiques ou CTL).

- Les lymphocytes B produisent des anticorps qui vont interagir avec les antigènes circulants de manière à les neutraliser.

- Enfin, les lymphocytes $T_8$ détruisent les cellules infectées quand ils reconnaissent les fragments d'antigènes associés au complexe majeur d'histocompatibilité de classe I.

La réponse immunitaire standard à un agent microbien quelconque peut être schématiquement résumée comme suit : l'intervention de l'immunité non-spécifique est quasiimmédiate puis, si besoin est, elle est suivie dans les 48 à 72 heures de l'apparition de la réponse immunitaire spécifique. Toutefois, il y a de notables exceptions, en particulier lorsque l'agent microbien est un virus. Dans ce cas-là , on observe parfois l'absence ou la difficile induction d'une réponse immunitaire spécifique.

L'absence d'induction pourrait s'expliquer par un effet suppresseur induit par l'agent viral dû à une surproduction de cytokines telles que l'IFN-α. Ces dernières pourraient empêcher ou freiner la mise en place de la réponse immunitaire.

Ce qui précède explique l'échec de certaines tentatives de vaccination. En effet, des éléments fortement immunogènes, candidats potentiels au titre de vaccin (virus ou bactérie inactivé ou atténué ; protéines d'origine virale, bactérienne ou parasitaire) se révèlent parfois insuffisants lors des essais in vivo, comme dans le SIDA ou l'Herpès.

Les infections à HIV (virus de l'immunodéficience humaine) constituent un cas typique de ce qui vient d'être évoqué. De manière toute particulière, on a constaté la présence d'IFN-α, et de néoptérine et de $\beta_2$ microglobuline marqueurs de la production d'IFN-α, en quantité significative dans le sang de patients présentant une infection à HIV (human immunodefficiency virus) tandis que le sang des individus sains est exempt d'IFN-α en quantité détectable ; on a également constaté la présence de corpuscules associés à la sécrétion d'IFN-α dans les ganglions d'individus infectés par HIV.

De plus, en culture in vitro de PBL (peripheral blood lymphocytes : lymphocytes + macrophages) issus de patients au stade ARC ou SIDA, la production d'IL-2 est sensiblement améliorée lorsque les PBL ont été traités par un sérum anti-IFN-α.

Une infection à HIV est détectée dans son tout premier stade par une séropositivité du patient qui persiste par la suite. De manière typique, cette infection évolue au cours des années, du stade asymptomatique au stade fulminant du syndrome d'immunodéficience acquise (SIDA) en passant par une lymphadénopathie généralisée et persistante (PGL) et un stade pré-SIDA ou SIDA-related complex (ARC). En parallèle à cette infection, se développent assez couramment des manifestations cliniques associées (ou infections opportunistes) tel les pneumonies à Pneumocystis carinii, des infections à cytomégalovirus, et des tumeurs tels que le sarcome de Kaposi. En résumé, une infection à HIV neutralise les défenses d'un individu en mettant en complète déroute son système immunitaire.

Le virus HIV a pour cible les cellules du système immunitaire exprimant le marqueur membranaire $CD_4$, essentiellement les cellules $T_4$ et les macrophages. Lors d'une infection, le nombre de lymphocytes $T_4$ circulants décroît progressivement aux stades PGL et ARC et chute brutalement au stade SIDA. Ainsi la perte sélective des lymphocytes $T_4$ et des macrophages résultant de l'activité cytostatique du HIV est directement le reflet du phénomène d'immunodéficience. Ce mécanisme peut reconnaître différentes étiologies dont l'apparition de facteurs de suppression.

Il serait également souhaitable disposer de dérivés anticytostatiques permettant de bloquer l'activité de ces facteurs de suppression.

On a maintenant trouvé que notamment un IFN-a, sous une forme immunogène et inactivée, ou un fragment ou un analogue inactif, était capable de stimuler la réponse immunitaire d'un individu et donc d'induire dans l'organisme une propriété antisuppressive à l'encontre des cellules de l'immunité (propriété dénommée anticytostatique).

C'est pourquoi la présente invention a pour objet une cytokine immunogène, inactivée ou un analogue inactif ou un fragment de cytokine.

Par "cytokine immunogène" on entend une cytokine capable d'induire une réponse immunitaire même dans l'espèce dont elle est issue, sachant que la cytokine native correspondante (cytokine substantiellement telle que trouvée dans une espèce) en est incapable ou très faiblement capable. Par exemple, une cytokine d'origine humaine administrée sous forme immunogène (forme différente de la forme native) à une humain, induit une réponse anti-cytokine qui se concrétise habituellement au moins par la production d'anticorps qui reconnaissent à la fois la cytokine administrée et la cytokine native. Parmi ces derniers, les anticorps neutralisants sont en particulier capables d'inhiber l'activité biologique de la cytokine native. Plus particulièrement, on considère qu'une cytokine immunogène est capable d'induire une réponse immunitaire humorale ou cellulaire chez une majorité d'individus de la même espèce.

Par "cytokine inactivée ou un analogue inactif" on entend une cytokine ou fragment de cytokine qui a perdu ou qui n'a pas d'activité biologique ; c'est à dire qui n'est pas capable d'agir en tant que régulateur de l'activité de la prolifération cellulaire.

Par "cytokine inactive", on entend un analogue d'une cytokine telle que trouvée dans une autre espèce, qui n'a pas d'activité biologique dans l'espèce à laquelle elle est administrée et dont la séquence en acides aminés diffère de la séquence naturelle par au moins un résidu acide aminé ; le degré d'homologie n'étant toutefois pas inférieur à 50% et de préférence pas inférieur à 80 %.

Par "cytokine inactivée" on entend une cytokine ou cytokine inactive qui a perdu son activité biologique et qui a été obtenue à partir d'une cytokine native (les cytokines natives dont on dispose sont le plus souvent produites par les techniques de génie génétique ayant subi notamment un traitement chimique, physique ou immunologique ou encore après encapsulation dans une émulsion dans des protéosomes ou des liposomes pour les inactiver).

Par "fragment inactif de cytokine", on entend un fragment d'une cytokine telle que trouvée dans une espèce, qui n'a pas d'activité biologique dans l'espèce à laquelle elle est administrée, notamment chez l'homme et dont la séquence ne acides aminés diffère de la séquence naturelle par au moins un résidu acide aminé ; le degré d'homologie n'étant toutefois pas inférieur à 50% et de préférence pas inférieur à 80%. Un tel fragment peut avoir de 4 à 100 acides aminés par exemple et de préférence de 6 à 60 acides aminés et notamment de 6 à 20. Il peut être fabriqué par synthèse peptidique ou par génie génétique (notamment bactéries ou cellules eucaryotes recombinantes).

De manière avantageuse, une cytokine selon l'invention est d'origine murine ou de préférence humaine.

Par "cytokine inactivée d'origine humaine" on entend une cytokine inactivée obtenue à partir d'une cytokine humaine.

De manière préférée, une cytokine selon l'invention est une cytokine immunogène et inactivée.

Une cytokine selon l'invention est de préférence sélectionnée parmi un IFN-$\alpha$, un IFN-$\beta$, un IFN-gamma, une interleukine (IL), notamment une IL-2, une IL-4, une IL-5, une IL-6, un TNF $\alpha$ ou $\beta$ et un TGF-$\beta$ et notamment un interféron-gamma, une interleukine-1, une interleukine-2, une interleukine-4, une interleukine-5, une interleukine-6, une interleukine-10 (IL-10) et un transforming growth factor-$\beta$ ou un fragment inactif ou inactivé desdites cytokines. On préfère plus particulièrement un TGF-$\beta$, un IFN-$\beta$ et un IFN-$\alpha$ selon l'invention ; ce dernier étant tout particulièrement préféré.

L'invention a aussi pour objet un procédé de préparation d'une cytokine immunogène et inactivée ou d'un fragment inactif de cytokine, qui comprend l'acte d'inactiver et de rendre immunogène une cytokine en la soumettant à un traitement immunologique, physique ou chimique. On notera qu'un seul et même traitement peut lui fait perdre son activité biologique et la rendre immunogène.

Un traitement physique approprié peut être un traitement par la chaleur ou des irradiations, une encapsulation (émulsion eau dans huile ou inclusion dans des vésicules tels les liposomes et les protéosomes). Un traitement chimique adapté peut être un traitement par un aldéhyde tel que le formaldéhyde ou un polyaldéhyde ou d'autres composés ($\beta$-propiolactone ou le polyéthylèneglycol par exemple). Un traitement au formaldéhyde est particulièrement préféré.

De manière typique, on traite 100 $\mu$g à 10 mg de cytokine/ml par du formaldéhyde ayant une concentration finale de 10mM à 100mM, à une température de 10 à 60°C, pendant 2 à 15 jours. Ces données sont uniquement fournies à titre indicatifs. Bien entendu chacun des paramètres varie en fonction des autres ; par exemple lorsque la concentration en formaldéhyde augmente, la durée de la réaction peut être réduite sans préjudice.

L'invention a aussi pour objet les cytokines ou fragments de cytokines sous forme inactivée et immunogène obtenus par la mise en oeuvre des procédés ci-dessus décrits, de préférence d'origine humaine notamment ceux ci-dessus décrits.

Comme on l'a vu, les cytokines, fragments ou analogues selon l'invention présentent de remarquables propriétés : ils sont notamment capables d'induire une réponse immunitaire de l'individu auquel il sont administrés (réaction de type humoral et cellulaire) à la manière d'un vaccin, mais agissant dans les cas d'hyperproduction de cytokines déréglant l'homéostasie, pour corriger ces dérèglements. Les produits selon l'invention présentent l'avantage de ne faire sentir leurs effets que lors de ces dérèglements.

Il n'induisent pas d'effets secondaires cliniques ou biologiques notables.

Au cours de l'évolution de l'infection à HIV, les individus produisent anormalement de l'IFN-$\alpha$, contribuant à un effet cytostatique sur les cellules de l'immunité. Des doses infimes d'IFN-$\alpha$ bloquent en effet la prolifération des cellules normales (telles que des LT de sang d'individus normaux) ; de plus des anticorps anti IFN-$\alpha$ ont partiellement restauré la prolifération et la production d'IL-2 par des LT de malades atteints du SIDA.

De même, la surproduction des autres cytokines contribue à induire d'autres états pathologiques :

- C'est le cas notamment de l'IL-4 qui est un cofacteur de stimulation des Lymphocytes B (LB) induisant la production d'IgE par lesdits LB. Ces IgE, par leur pouvoir de dégranulation des basophiles et des mastocytes, contribuent aux manifestations pathologiques liées à l'allergie.

- C'est le cas aussi par exemple de l'IL-6 qui contribue à la sécrétion d'Ig (immunoglobulines) par les cellules cancéreuses de myélome.

L'administration de cytokines, fragments ou analogues selon l'invention produit une réaction immunitaire de l'organisme restaurant l'équilibre compromis grâce à la réaction immunitaire humorale et cellulaire (anticorps ou cellules tueuses anti-cytokine concernée).

Les cytokines, fragments ou analogues selon l'invention trouvent de ce fait des applications selon la cytokine concernée, tant curatives que préventives notamment dans le traitement du SIDA pour l'IFN-$\alpha$, dans le traitement de l'allergie pour l'IL-4 et l'IL-5, dans le traitement de certaines affections auto-immunes (Lupus Erythémateux, Polyarthrite rhumatoïde, Sclérodermie ...), le traitement de certaines maladies cancéreuses telles que le mélanome multiple pour l'IL-6, et plus généralement les autres maladies sous-tendues par le dérèglement en excès de cytokines (incluant comme on l'a vu les neuropeptides comme la somatostatine)

Les dérivés selon l'invention ont une très faible toxicité tant chez l'animal que chez l'homme. Des doses de 20 mg administrées à la souris aux jours 0, +21, +41 n'ont produit aucune toxicité. Une dose de 200 à 400 mg injectée à l'homme par voie intramusculaire de dérivé de l'exemple 1 par exemple n'a produit aucun effet toxique décelable.

Ces propriétés justifient l'application des cytokines, fragments ou analogues selon l'invention à titre de médicaments.

C'est pourquoi la présente invention a aussi pour objet :

a) Une composition pharmaceutique comprenant à titre d'agent thérapeutique, une cytokine ou fragment ou analogue de cytokine selon l'invention.

b) Une composition pharmaceutique destinée au traitement d'une tumeur maligne telle qu'un myélome multiple dont la sécrétion d'Ig est liée à l'IL-6, d'une affection auto-immune ou d'une allergie (urticaire, rhinites allergiques) associées à l'hyperproduction d'Ig, liée à la surproduction d'IL-4 ou IL-5 qui comprend à titre d'agent thérapeutique une cytokine, fragment ou analogue selon l'invention sélectionnée parmi une IL-6, une TGF-$\beta$, une IL-1, une IL-2, une IFN-gamma, une IL-5 et une IL-4.

c) Une composition pharmaceutique destinée à la stimulation des défenses immunitaires d'un individu, qui comprend à titre d'agent thérapeutique une cytokine, fragment ou analogue selon l'invention sélectionnée parmi un TGF-$\alpha$, une IFN-$\beta$, un TNF et un IFN-$\alpha$ selon l'invention.

d) Une composition pharmaceutique destinée au traitement d'une infection à HIV de préférence à HIV-1, qui comprend à titre d'agent thérapeutique un IFN-$\alpha$, fragment ou analogue d'IFN-$\alpha$ selon l'invention.

Ces compositions pharmaceutiques comprennent habituellement un excipient pharmaceutique acceptable en plus d'une quantité thérapeutiquement efficace du principe actif.

Une composition pharmaceutique telle que répertoriée sous d) est d'un intérêt tout particulier lorsqu'il s'agit de traiter une infection à HIV de manière prophylactique thérapeutique au stade ARC ou SIDA ou des manifestations cliniques associées. En effet, un IFN-$\alpha$ selon l'invention a une activité anti-cytostatique à l'encontre des lymphocytes $T_4$ des patients et ainsi favorise le maintien, sinon la croissance du nombre des lymphocytes $T_4$, avec pour conséquence directe le maintien ou la reprise de l'activité des lymphocytes T cytotoxiques. Ceci peut être mis en évidence dans un test CMI (immunité à médiation cellulaire) tel que décrit dans l'exemple 4 ci-après.

Une composition pharmaceutique répertoriée sous c) ou d) peut en outre contenir un agent vaccinal conventionnel. Par "agent vaccinal conventionnel" on signifie :

(i) Un agent pathogène traité ou modifié pour le rendre noninfectieux ; ceci peut être par exemple une bactérie ou un virus entier qui a perdu son caractère infectieux grâce à un traitement chimique (agent vaccinal inactivé) ou dont la virulence a été atténuée par mutagénèse ou par passage dans un milieu qui provoque des mutations (agent vaccinal tué) ;

(ii) Un déterminant antigénique caractéristique d'un agent pathogène, sous forme isolée dudit agent pathogène (agent vaccinal sous-unité) ; ceci peut être par exemple une toxine bactérienne ou un fragment de celle-ci, un antigène spécifique d'un agent infectieux tel qu'un polyoside capsulaire ou un fragment de celui-ci, une protéine virale par exemple d'enveloppe ou de capside ou un fragment de celle-ci et un antigène parasitaire ou un fragment de celui-ci, cette liste étant toutefois non limitative ; ou

(iii) Un déterminant antigénique caractéristique d'une tumeur.

En outre, l'invention propose également un kit comprenant :

a) Une composition pharmaceutique destinée à la stimulation des défenses immunitaires d'un individu, qui comprend à titre d'agent thérapeutique une cytokine, fragment ou analogue selon l'invention sélectionnée parmi un TGF-$\beta$, un IFN-$\beta$ et un IFN-$\alpha$ selon l'invention ou encore un TNF
b) Une composition pharmaceutique vaccinale conventionnelle, et
c) Des instructions pour l'administration concomitante ou consécutive des compositions a) et b).

Par "composition vaccinale conventionnelle" on entend une composition qui comprend un agent vaccinal conventionnel, c'est à dire au moins un antigène spécifique de l'agent pathogène contre lequel la vaccination est dirigée.

Une composition pharmaceutique selon l'invention peut en outre comprendre un adjuvant. D'une manière générale, la présence d'adjuvant permet de diminuer la dose de l'agent thérapeutique par rapport à une composition qui n'en contiendrait pas.

Enfin, une composition pharmaceutique selon l'invention peut être fabriquée sous une forme galénique conventionnelle. En particulier on associe une cytokine selon l'invention en quantité suffisante pour être efficace d'un point de vue thérapeutique, avec un diluant ou un support acceptable d'un point de vus pharmaceutique. Une composition selon l'invention peut être administrée par n'importe quelle voie conventionnelle en usage dans le domaine des vaccins, en particulier par voie sous-cutanée, par voie intramusculaire ou par voie intraveineuse , par exemple sous forme de suspension injectable.

L'administration peut avoir lieu en dose unique ou répétée une ou plusieurs fois après un certain intervalle. Le dosage approprié varie en fonction de divers paramètres, par exemple, de l'individu traité ou du mode d'administration. A titre d'exemple, on indique toutefois que, chez des patients présentant une infection à HIV, on obtient des résultats satisfaisants avec une dose d'IFN-$\alpha$ selon l'invention de 1 à 50 $\mu$g/kg de mammifère, de manière avantageuse de 3 à 20 $\mu$g/kg de mammifère, de préférence de 3 à 8 $\mu$g/kg de mammifère. Les doses les plus faibles sont avantageusement administrées en présence d'adjuvants et/ou à intervalles répétés tandis que les doses les plus fortes peuvent être administrées une seule fois et/ou en absence d'adjuvant.

De même, l'invention a pour objet :

e) L'usage d'une cytokine fragment ou analogue selon l'invention à des fins thérapeutiques ; en particulier, l'usage d'une IL-6, d'un TGF-$\beta$, d'une IL-1, d'une IL-2, d'un IFN-gamma, d'une IL-4 ou d'une IL-5 selon l'invention pour le traitement d'une tumeur, d'une affection auto-immune ou d'une allergie ; de manière toute particulière, l'usage d'un TGF-$\beta$, d'un IFN-$\beta$ ou d'un IFN-$\alpha$ selon L'invention pour stimuler les défenses immunitaires d'un individu par exemple pour renforcer le pouvoir vaccinal d'un agent vaccinal conventionnel ; de manière préférée, l'usage d'un IFN-$\alpha$ selon l'invention pour traiter une infection virale par virus à enveloppe, notamment à HIV.

f) Une méthode de traitement thérapeutique qui comprend l'acte d'administrer une cytokine, fragment ou analogue selon l'invention en quantité suffisante pour être efficace d'un point de vue thérapeutique, à un sujet ayant besoin d'un tel traitement.

EP 0 591 281 B1

g) L'utilisation d'une cytokine, fragment ou analogue selon l'invention pour préparer une composition pharmaceutique destinée au traitement d'une tumeur maligne et/ou d'une maladie auto-immune et/ou d'une allergie et/ou à la 5 stimulation des défenses immunitaires et/ou traitement d'une infection par virus à enveloppe particulièrement à HIV, notamment obtenus par le procédé de préparation défini plus haut.

On a aussi trouvé que dans le cas d'une infection à virus à enveloppe, notamment à rétrovirus, l'effet immunosuppresseur pourrait être non seulement attribué à une hyperproduction de cytokines telles que l'IFN-α dans le cas de l'infection à HIV-1 par les cellules infectées mais aussi au blocage de l'activation des LT (phénomène d'anergie) par des composants peptidiques du rétrovirus lui-même. Ces composants agissent soit directement, soit par homologie avec le site d'une substance immunorégulatrice (mimétisme ou inhibition) c'est le cas pour le peptide identifié par l'identificateur de séquence (ci-après IS) N° 6 ci-après.

Plus particulièrement, certains sites responsables de cette activité inhibitrice ont été localisés notamment sur les sous-unités de protéines env, (gp 120 et gp 41) et gag des virus à enveloppe.

En conséquence, l'invention propose un peptide ayant une activité cytostatique à l'encontre des cellules du système immunitaire comprenant une séquence d'acides aminés substantiellement identique à la séquence d'une région d'une des sous-unités de la protéine gag ou env d'un virus à enveloppe ; ladite séquence d'une région d'une des sousunités de la protéine ci-dessus contenant ou étant adjacente à un peptide ayant au moins 50%, de manière avantageuse au moins 70%, de manière préférée au moins 80%, de manière tout à fait préférée au moins 90% d'homologie avec la séquence d'un segment actif d'une protéine humaine ayant une activité immunorégulatrice, notamment cytostatique.

Par "segment actif", on entend le site portant l'activité, ou bien ledit site ainsi que les sites qui lui sont adjacents, ou encore les sites adjacents dans la mesure où les anticorps dirigés contre ces sites adjacents bloquent fonctionnellement le site portant l'activité.

Par "séquence substantiellement identique à une autre séquence", on signifie une séquence qui présente un degré d'homologie d'au moins 50%, avantageusement d'au moins 90%, de préférence d'au moins 95% avec l'autre séquence.

Les peptides selon l'invention sont présentés sous forme immunogène, et dénués d'activité immunosuppressive.

Un peptide préféré selon l'invention comprend :

(i) Une séquence d'acides aminés substantiellement identique à la séquence d'une région d'une des sous-unités de la protéine gag ou de la protéine env d'un rétrovirus notamment les virus HIV-2 et HIV-1, particulièrement ce dernier, cette séquence contenant elle-même un peptide ayant une homologie de préférence d'au moins 50%; ou
(ii) une séquence d'acides aminés substantiellement identique à la séquence d'une région d'une des sous-unités de la protéine gag ou de la protéine env d'un virus HIV.

Un peptide selon l'invention contient de manière avantageuse au moins 5 acides aminés et au plus 50 acides aminés, de préférence au plus 30 acides aminés.

On retient notamment un peptide selon l'invention, qui comprend une séquence d'acides aminés substantiellement telle que montrée dans l'identificateur de séquence (IS) N°1a, 2a, 3a ou 4. On retient également ceux montrés dans les IS N° 7, 8, 11, 12 et notamment 5, 6, 9 et 10.

Les peptides peuvent être utilisés directement dans des émulsions eau dans huile ou enchâssés au sein d'adjuvants aqueux de phosphate de calcium ou encore adsorbés dans un phosphate d'alumine. Les peptides peuvent aussi être présentés conjugués à des protéines porteuses, comme le tétanos toxoïde ou encore le KLH. Enfin, les peptides peuvent également être présentés au sein de bactéries dans des constructions de recombinaison génétique, après l'insertion de séquences nucléotidiques appropriées dans des plasmides.

Certains peptides non immunogènes et immunosuppresseurs sont nouveaux, notamment ceux montrés dans les IS 6 à 12, notamment IS 6 et 7. Ces peptides font aussi l'objet de l'invention.

Une composition pharmaceutique selon l'invention est avantageusement destinée à la prévention ou au traitement d'une infection à HIV, plus particulièrement destinée au traitement d'une infection à HIV au stade ARC ou SIDA.

L'immunogénicité d'un peptide selon l'invention peut être conférée par la préparation de protéosomes ou par l'utilisation d'un composé lipidique acceptable d'un point de vue pharmaceutique qui a de préférence les propriétés d'un adjuvant.

Par exemple, on peut préparer une composition selon l'invention en diluant un peptide selon l'invention dans une suspension eau/huile. D'autre part, des protéosomes appropriés sont tels que décrits et obtenus dans Lowell et al, J.Exp.Med., (1988) 167 : 658. Lorsqu'un peptide selon l'invention est associé à des protéosomes, il est de préférence préparé sous forme de conjugué, c'est à dire qu'il est lié par liaison covalente à un groupement hydrophobe tel qu'un radical acide gras, par exemple un radical lauryl ou lauryl-Cys, ou bien il comprend outre la séquence caractérisante, une séquence-pied composée d'acides aminés hydrophobes par exemple le pied Phe-Leu-Leu-Ala-Val-Phe-Cys; la séquence-pied est de préférence liée par liaison peptidique à l'extrémité $NH_2$-terminale de la séquence caractérisante.

Les produits ci-dessus sont dotés de remarquables propriétés qui justifient leur utilisation comme médicament.

- Les peptides natifs (isolés ou obtenus par synthèse ou génie génétique) peuvent être utilisés pour induire une immunosuppression ; une telle propriété est illustrée ci-après dans la partie expérimentale pour les IS N° 5, 6, 7 et 10.
Inactivés et rendus immunogènes ils peuvent être utilisés pour provoquer dans l'organisme considéré une réaction immunitaire, cellulaire et humorale neutralisant les effets biologiques de ces peptides.

- Les peptides sous forme inactivée mais immunogènes pourront être utilisés comme ci-dessus.
    C'est pourquoi la présente demande a aussi pour objet les médicaments caractérisés en ce qu'ils sont constitués par les peptides natifs ou modifiés définis ci-dessus, ainsi que les compositions pharmaceutiques les renfermant. Les médicaments ci-dessus peuvent être utilisés, pour ce qui concerne les peptides immunogènes, à titre de traitement pour créer une immunisation active vis à vis d'agents pathogènes les renfermant : par exemple vis à vis des glycoprotéines gp 160, 120 ou 41 de HIV-1 qui contient par exemple le site ILAVERY homologue au site actif de l'IFN-$\alpha$, ou le site LERILL correspondant au site de liaison de l'IL-2 avec son récepteur (IL-2R).

De même :

- le peptide SLWDQ est totalement identique entre le HIV-1 enveloppe (résidus 110 à 114) et le CD4 (résidus 60 à 64). Voir IS N° 6
- le peptide CTASQK de la molécule CD4 (résidus 16-21) partage une forte homologie avec enveloppe du HIV-1 (CSATEK, résidus 28 à 33). Voir IS N° 7
- Le peptide EPTAPP est commun entre le CD5 et la protéine gag du HIV-1, et unique à ces deux molécules parmi toutes les protéines connues. Il correspond au résidus 454-459 du HIV-1 gag et aux résidus 153-158 du récepteur CD5. Voir IS N° 8
- Le peptide TTLFCA (résidus 20 à 25) de l'enveloppe du HIV-1 a une très forte homologie avec le peptide TTLFCL du TNF alpha (résidus 45 à 50). Voir IS N° 9
- Le peptide LLLNGSLA de l'enveloppe du HIV-1 (résidus 259-267) possède une similitude importante avec les molécules régulatrices suivantes : HLA de classe I (résidus 11-18), chaîne $\beta$ du récepteur des cellules T (résidus 14 à 21), et G-CSF (résidus 17 à 24). Voir IS N° 10.
- Le peptide QLTVW est commun à l'enveloppe du HIV-1 (résidus 566-570) et à la chaîne du récepteur des cellules T (résidus 130-134). Voir IS N° 11.
- Le peptide GIRPVV de l'enveloppe du HIV-1 (résidus 250-256) a une très forte homologie avec le peptide GIRKVV de la chaîne a du précurseur de la protéine d'adhésion leucocytaire (résidus 230-236). Voir IS N° 12.

De manière similaire, on peut aussi créer une immunisation vis à vis de peptides issus de site actifs d'autres virus à enveloppe induisant une immunosuppression, notamment ceux de la famille de l'Herpès, HIV-2, HTLV1 et le virus de la grippe.

En ce qui concerne les peptides actifs, immunosuppresseurs non inactivés, ils peuvents être utilisés notamment dans le traitement du rejet de greffes.

Il peuvent être administrés à titre curatif ou préventif.

Les peptides immunogènes ont une très faible toxicité ; administrés à la dose de 20 µg à la souris, aucune toxicité n'a été observée.

Une composition pharmaceutique selon l'invention peut en outre contenir un agent conventionnel anti infectieux, destiné à vacciner contre une infection à rétrovirus, notamment HIV. Pour usage à l'encontre d'une infection à HIV, un agent vaccinal conventionnel avantageux est par exemple la protéine env sous la forme de la protéine gp160 non-clivable, la sous unité gp 120 de la protéine env, un fragment de la gp120 comportant la boucle $V_3$ ou des virions HIV déplétés de leur ARN génomique.

Enfin, une composition pharmaceutique vaccinale selon l'invention peut être fabriquée de manière conventionnelle. En particulier, on associe un peptide selon l'invention en quantité suffisante pour être efficace d'un point de vue thérapeutique, avec un solvant ou un support acceptable d'un point de vue pharmaceutique. Une composition selon l'invention peut être administrée par n'importe quelle voie conventionnelle en usage dans le domaine des vaccins, en particulier par voie sous-cutanée, par voie intramusculaire, par voie intraveineuse ou par voie intradermique, par exemple sous forme de suspension injectable, ou encore par voie orale. L'administration peut avoir lieu en dose unique ou répétée une ou plusieurs fois après une certain intervalle. Le dosage varie en fonction de divers paramètres, par exemple, de l'individu traité ou du mode d'administration. A titre d'exemple, on indique toutefois que, chez des patients présentant un infection à HIV, on obtient des résultats satisfaisants avec une dose de chacun des peptides des IS N° 1a, 2a, 3a, 4, 5, 6 et 7 à 20 μg/Kg de poids corporel, de manière avantageuse de 4 à 16 μg/kg de poids corporel, de préférence de 8 à 12 μg/Kg de poids corporel, tant présentés sous forme de protéosomes que couplés à des toxoïdes ou qu'émulsionnés.

Les doses les plus faibles sont avantageusement administrées en présence d'adjuvants et/ou à intervalles répétés tandis que les doses les plus fortes peuvent être administrées une seule fois et/ou en absence d'adjuvant. De même l'invention alternative propose :

(i) Un kit contenant :

a) Une composition pharmaceutique selon l'invention alternative,
b) Une composition pharmaceutique vaccinale conventionnelle, et
c) Des instructions pour l'administration concomitante ou consécutives des compositions a) et b).

(ii) L'usage d'un peptide selon l'invention pour prévenir ou traiter un infection à virus à enveloppe, particulièrement à rétrovirus, notamment à HIV.
(iii) Une méthode pour prévenir ou traiter une infection à virus à enveloppe, particulièrement à rétrovirus, notamment à HIV, qui comprend l'acte d'administrer un peptide selon l'invention en quantité suffisante pour être efficace d'un point de vue thérapeutique et en association avec un élément capable d'inhiber l'activité potentiellement cytostatique dudit peptide, à un sujet ayant besoin d'un tel traitement.
(iv) L'utilisation d'un peptide selon l'invention pour préparer une composition pharmaceutique destinée à la prévention ou au traitement d'une infection à virus à enveloppe, particulièrement à rétrovirus, notamment à HIV, notamment au stade ARC ou SIDA notamment par le procédé de fabrication défini plus haut.

L'invention concerne également un virus déplété de son génome à savoir qu'il ne contient plus d'acide nucléique génomique dudit virus. On préfère les virus à ARN déplétés dudit ARN, notamment ceux à enveloppe.
Un virus déplété de son génome selon l'invention dérive avantageusement d'un virus à enveloppe, notamment d'un rétrovirus, de préférence d'un virus HIV.
De même l'invention propose un procédé de préparation d'un virus déplété de son génome, qui comprend :

a) L'acte de soumettre un virus à une hydrolyse alcaline (pour virus à ARN) ou acide (pour virus à ADN), et
b) l'acte de récolter un virus déplété de son génome obtenu en a).

Sous un aspect plus particulier, un procédé selon l'invention, peut être notamment mis en oeuvre selon l'alternative suivante.
La première comprend :

a) l'acte de soumettre un virus à une hydrolyse alcaline,
b) l'acte de traiter un virus déplété de son génome obtenu en a) par un aldéhyde et,
c) L'acte de récolter le virus déplété de son génome obtenu en b).

La deuxième comprend :

a) l'acte de traiter un virus par aldéhyde,
b) l'acte de soumettre le virus obtenu en a) à une hydrolyse alcaline, et
c) l'acte de récolter un virus déplété de son génome obtenu en b).

Il en est de même pour l'hydrolyse acide. Un aldéhyde avantageux est le formaldéhyde ou un polyaldéhyde comme le glutaraldéhyde ; ces deux derniers étant préférés. L'hydrolyse alcaline destinée à dépolymériser l'ARN peur être notamment mise en oeuvre en présence de n'importe quel composé qui hydrolyse l'ARN ; par exemple de l'hydroxyde de sodium ou de l'hydroxyde de potassium, ce dernier étant préféré. Il en est de même pour l'hydrolyse acide. Comme acides on peut citer notamment l'acide acétique. Les virus déplétés selon l'invention sont dotés de remarquables propriétés. Ils sont notamment immunogènes en induisant une réponse immunitaire humorale et cellulaire ; une telle propriété est illustrée ci-après dans la partie expérimentale. Ils ont de plus perdu leur pouvoir infectieux. Ces propriétés justifient leur application à titre préventif ou curatif comme médicament.
Les virus déplétés selon l'invention ne présentent pas de toxicité décelable chez l'homme ou chez l'animal.
Ils peuvent être utilisés notamment dans le traitement des affections virales : notamment à HIV-1, HIV-2, HTLV-1, FELV, FIV et les autres infections par virus à enveloppe notamment ceux de la famille de l'Herpès.

Une composition selon l'invention destinée à la prévention d'une infection à rétrovirus est une composition vaccinale dans laquelle un virus déplété de son génome selon l'invention est présent à titre d'immunogène.

Une composition pharmaceutique selon l'invention peut en outre comprendre un adjuvant. D'une manière générale, la présence d'adjuvant permet de diminuer la dose de l'agent thérapeutique par rapport à une composition qui n'en contiendrait pas.

Enfin, une composition pharmaceutique selon l'invention peut être fabriquée de manière conventionnelle. En particulier, on associe un peptide selon l'invention, en quantité suffisante pour être efficace d'un point de vue thérapeutique, avec un solvant ou un support acceptable d'un point de vue pharmaceutique.

Une composition selon l'invention peut être administrée par n'importe quelle voie conventionnelle en usage dans le domaine des vaccins, en particulier par voie sous-cutanée, par voie intramusculaire ou par voie intraveineuse, par exemple sous forme de suspension injectable. Elle peut aussi être administrée par voie orale. L'administration peut avoir lieu en dose unique ou répétée une ou plusieurs fois après un certain intervalle. Le dosage approprié varie en fonction de divers paramètres, par exemple de l'individu traité ou du mode d'administration. A titre d'exemple, on indique toutefois que, chez des patients présentant une infection à HIV, on obtient des résultats satisfaisants par exemple avec une dose de virus HIV déplété de son génome de 10 à 40 µg/kg de poids corporel, de manière avantageuse de 20 à 30 µg/kg de poids corporel. Les doses les plus faibles sont avantageusement administrées en présence d'adjuvants et/ou à intervalles répétés tandis que les doses les plus fortes peuvent être administrées une seule fois et/ou en absence d'adjuvant.

L'invention est illustrée ci-après :

## EXEMPLE 1 :

### Inactivation d'une IFN-$\alpha$ en présence de formaldéhyde.

A 8 ml d'une solution d'IFN-$\alpha$ à 1 mg de protéine/ml (Hoffmann-Laroche), on ajoute du phosphate de sodium 70mM final et du formaldéhyde 33mM final. L'incubation est poursuivie 3 jours à 37°C sans agitation. On ajoute de la lysine 1mg/ml final.

La préparation est ensuite dialysée contre du tampon PBS pH 7,2 dilué 10 fois (phosphate buffer saline ; 20 mM de phosphate de sodium et 150 mM de chlorure de sodium) pendant 3-4 heures à 4°C. Puis le dialysat est réparti en 20 doses de volume identique et chacune des doses est lyophilisée.

## EXEMPLE 2

### Formulation de peptides en protéosomes.

Les peptides ayant une séquence telle que montrée dans les IS N° 1b à 3b et 5 ont été synthétisés par Neosystem (Strasbourg, France) et préparés séparément sous forme de lyophilisats.

Chacun des lyophilisats est repris dans du tampon TE (50mM Tris pH 7,4 ; 0,5mM EDTA) à 1% de détergent Empigen BB (Albright & Wilson, Whithaven, Cumbria) pour obtenir une solution à 2mg/ml de peptide.

On mélange ensemble des quantités égales de chacune des solutions peptidiques auxquelles on ajoute par la suite une solution de protéosomes préparée selon la méthode de Lowell et al, J.Exp.Med. (1988) 167 : 658, dans un rapport peptides : protéosomes de 8 : 1 (poids/poids).

Cette préparation est dialysée contre du PBS à 4°C pendant 10 jours dans des conditions stériles. Après dialyse, la préparation contient environ 200 µg/ml de protéosomes et environ 800 µg/ml de peptides fixés dans les protéosomes.

## EXEMPLE 3

### Préparation d'une composition pharmaceutique à base d'IFN-$\alpha$ inactivé et de peptides.

3A) Composition N°1

400 µl d'une solution de phosphate de calcium 35mM et de chlorure de sodium 35mM, pH 7,2 sont centrifugés. Le surnageant est éliminé. Le dépôt de phosphate de calcium est repris dans 400 µl de la préparation telle qu'obtenue à l'exemple 2.

Enfin ces 400µl sont utilisés pour dissoudre une dose telle qu'obtenue à l'exemple 1 (contenant 400 µg ± 200 µg d'IFN-$\alpha$ inactivé).

3B) Composition N° 2

Une dose telle qu'obtenue à l'exemple 1 et 500 µg du lyophilisat de chacun des peptides ayant une séquence telle que montrée dans les IS N° 1b à 3b et 5 sont mélangés ensemble et repris dans 1 ml d'une suspension eau/huile minérale ref. ISA 724 (Seppic, France).

## EXEMPLE 4

**Traitement de patients présentant une infection à HIV au stade ARC avec de l'IFN-α inactivé et/ou des peptides.**

Quatre Patients K11, K41, K80 et K711 présentant une infection à HIV au stade ARC sont médicalement suivis depuis Novembre-Décembre 1986 (K11, K41 et K80) ou depuis Janvier 1989 (K711). Ils sont en particulier soumis à une immunothérapie qui consiste en des injections répétées à des intervalles variant de 1 à 8 mois, de PBL (peripheral blood lymphocytes = lymphocytes + macrophages) autologues infectés en culture in vitro par du virus HIV-1. La dernière injection de cellules autologues a lieu en Mai 1990.

A partir d'Octobre 1990, les patients sont soumis à une nouvelle immunothérapie dont le protocole est indiqué dans le tableau ci-dessous.

|  | K11 | K41 | K80 | 4711 |
|---|---|---|---|---|
| OCTOBRE 90 | P | P | P+I | P+I |
| FEVRIER 91 | P | P+I | P+I | P+I |
| AVRIL 91 | P | P+I | P+I | P+I |

"P" représente une dose de 500 µg de chacun des peptides ayant une séquence telle que montrée dans les IS N° 1b à 3b et 5.

"I" représente une dose de 400 µg ± 200 µg d'IFN-α.

"P" et P+I" sont injectés sous forme d'une composition telle qu'obtenue à l'exemple 3A ou 3B.

L'amélioration de l'immunité de chacun des patients est suivie en mesurant la quantité de lymphocytes $T_4$ (T-auxilliaires) par $mm^3$ de sang (pour mémoire on indique que chez individu sain, la concentration normale en lympho-cytes $T_4$ est de l'ordre de 500 à 1000 lymphocytes par $mm^3$ de sang) ainsi que par l'évaluation de la réponse immunitaire à médiation cellulaire in vivo et in vitro.

L'évaluation in vivo s'effectue grâce à un test intradermique à la candidine ou à la tuberculine (Pasteur-Mérieux).

L'évaluation in vitro s'effectue par test CMI ; ce dernier étant mis en oeuvre comme suit :

Le sang prélevé est dilué au 1/3 en milieu RPMI 1640. Les cellules mononuclées sont isolées par centrifugation en gradient de Ficoll-Hypaque 25 minutes à 1800 tours/ min. L'interface du gradient est récupérée et lavée 2 fois en milieu RPMI 1640 (centrifugation à 1500 tours / min. pendant 10 minutes). L'élimination totale des globules rouges est contrôlée par numération cellulaire en acide acétique 1% et on s'assure de la viabilité des cellules mononuclées par une numération bleu trypane.

Les cellules mononuclées sont reprises en milieu RPMI 1640 complémenté à 10% de sérum humain.

Des aliquots de cette préparation sont distribués dans les puits à fond rond d'une plaque de micro-culture à raison de $2.10^5$ cellules/puits. On ajoute une quantité optimale d'antigène à tester c'est à dire candidine ou tuberculine (quantité optimale à déterminer suivant les lots). Le tout est sou un volume de 200 µl/puits.

La culture cellulaire est poursuivie à 37°C en atmosphère humide chargée à 5% de $CO_2$ pendant 6 jours. 18 heures avant la fin de l'incubation, on ajoute 0,5 µCi de thymidine tritiée ayant une activité spécifique de 2 µCi/mole.

Les cellules sont ensuite récoltées sur filtre, lavées et séchées. La radioactivité β est enfin mesurée.

L'intensité de la réponse immunitaire est proportionnelle à l'index de prolifération $I_p$.

$$I_p = \frac{\text{radioactivité mesurée pour l'antigène donné}}{\text{radioactivité mesurée pour témoin négatif}}$$

Dans le tableau ci-après, on présente l'ensemble des résultats.

|  |  | K11 | K41 | K280 | K711 |
|---|---|---|---|---|---|
| 1. Lymphocyte $T_4$/mm$^3$ | Mai 90 | 240 | 273 | 187 | 476 |
|  |  | 29% | 13% | 11% | 31% |
| 2. % Lymphocytes $T_4$ (par rapport à l'ensemble des cellules mononuclées) | Avril 91 | 247 | 440 | 348 | 1000 |
|  |  | 22% | 14% | 13% | 40% |
| Test de réaction intradermique | Mai 90 | - | - | - | - |
| 1. candidine |  | - | - | - | - |
| 2. tuberculine | Avril 91 | - | ++ | + | ++ |
|  |  | + | ++ | + | +++ |
| Test CMI | Mai 90 | ND | ND | ND | ND |
| 1. candidine |  | ++ | + | + | ++++ |
| 2. tuberculine | Avril 91 | +++ | + | + | ++++ |

Ces résultats révèlent une augmentation sensible de la population des lymphocytes $T_4$ entre Mai 90 et Avril 91 ainsi qu'une amélioration notable de la capacité du système immunitaire des patients à réagir à l'encontre d'un antigène.

**EXEMPLE 5**

**Production d'une suspension de virus déplétés de leur génome HIV-1 à partir de virions HIV-1.**

5A) Préparation N°1

Le surnageant d'une culture de la lignée de lymphocytes T4 H9/IIIB ou MN infecté par le virus HIV1, souche de HTLV IIIB, est prélevé puis centrifugé à 40,000 xg à 4°C pendant 2 heures. Le culot est resuspendu dans 1 à 2 ml de tampon PBS pH 7,2. La suspension est centrifugée dans les mêmes conditions que précédemment. Le culot est à nouveau repris dans 100 ml de PBS. La concentration en protéines totales de la suspension ainsi obtenue est de l'ordre de 2 mg/ml telle que déterminée par la méthode de Lowry. La concentration en virions de la suspension est estimée devoir correspondre à environ 50% de la concentration en protéines totales. Cette estimation repose sur une analyse par Western blot.
A 10 ml de la suspension obtenue, on ajoute de l'hydroxyde de potassium 0,3 N final. On incube le mélange une nuit à 37°C sans agitation. puis on neutralise la préparation avec de l'acide chlorhydrique.
A cette préparation, on ajoute ensuite du phosphate de sodium 70 mM final pH 7,2 et du formaldéhyde 33 mM final. A ce stade, la préparation contient environ 1 mg/ml de protéines totales. Cette préparation est incubée 3 jours à 37°C sans agitation.
Puis on ajoute du glutaraldéhyde 0,5 mM final et on laisse incuber 30 minutes à température ambiante. Enfin, on ajoute de la lysine 1 mg/ml final. Le mélange est dialysé contre du PBS dilué dix fois, 3-4 heures à 4°C. Le dialysat est ensuite complètement lyophilisé.

5B) Préparation 2

10 ml d'une préparation de virus HIV-1 en tampon PBS dont la concentration, en protéines est de l'ordre de 1 mg/ml est traitée par le formaldéhyde selon la méthode reportée à l'exemple 5A. Après addition de lysine, on procède au traitement par l'hydroxyde de potassium comme décrit à l'exemple 5A. Après neutralisation , la préparation est dialysée contre du PBS dilué 10 fois, 3-4 heures à 4°C. Le dialysat est finalement complètement lyophilisé.

## EXEMPLE 6

### Composition pharmaceutique à base de virus déplété de son génomes HIV-1

6A) Composition N° 1

Le lyophilisat obtenu à l'exemple 5A ou 5 B est repris dans 400 µl d'eau distillée stérile apyrogène, puis mélangé à 600 µl du mélange eau/huile ISA 724.

6B) Composition N° 2

Le lyophilisat obtenu à l'exemple 5A ou 5B, une dose d'IFN-$\alpha$ telle que obtenue dans l'exemple 1, ainsi que 500 µg du lyophilisat de chacun des peptides ayant une séquence montrée dans les IS N° 1b à 3b et 5 sont repris dans 400 µl d'eau distillée stérile apyrogène, puis mélangé à 600 µl du mélange eau/huile ISA 724.

EXEMPLE 7

### Traitement des patients présentant une infection à HIV au stade ARC avec en particuliers des virus déplété de son génomes HIV-1.

Deux patients K280 et G210 présentant une infection à HIV au stade ARC sont médicalement suivis depuis Juillet 1986 et Janvier 1990 respectivement. Ils sont en particulier soumis à une immunothérapie qui consiste en des injection répétées à des intervalles variant de 1 à 8 mois, de PBL autologues infectés en culture in vitro par du virus HIV-1. La dernière injection de cellules autologues a lieu en Mai 1990.

A partir d'Octobre 1990, les patients sont soumis à une nouvelle immunothérapie dont le protocole est indiqué dans le tableau ci-dessous.

|  | K280 | G210 |
|---|---|---|
| OCTOBRE 90 | S | S |
| FEVRIER 91 | S+P+I | S+P+I |
| AVRIL | S+P+I | S+P+I |

"P" représente une dose de 500 µg de chacun des peptides ayant une séquence telle que montrée dans les IS N° 1b à 3b et 5.

"I" représente une dose de 400 µg ± 200 µg d'IFN-$\alpha$.

"S" représente une dose de la composition de virus déplété de son génome telle qu'obtenue à l'exemple 6A correspondant à environ 2 mg de protéine.

"S+P+I" sont injectés sous forme d'une composition telle qu'obtenue à l'exemple 6B.

L'amélioration de l'immunité de chacun des patients est suivie en mesurant la quantité de lymphocytes $T_4$ (T-auxilliaires) par $mm^3$ de sang (pour mémoire, on indique que chez un individu sain, la concentration normale en lymphocytes $T_4$ est de l'ordre de 500 à 1000 lymphocytes $T_4$ par $mm^3$ de sang) ainsi que par l'évaluation de la réponse immunitaire à médiation cellulaire in vivo et in vitro.

L'évaluation in vivo s'effectue grâce à un test intradermique à la candidine ou à la tuberculine (Pasteur-Mérieux).

L'évaluation in vitro s'effectue par test CMI ; ce dernier étant mis en oeuvre comme décrit à l'exemple 4.

Dans le tableau ci-dessous, on présente l'ensemble des résultats.

|  |  | K280 | G210 |
|---|---|---|---|
| 1. Lymphocyte $T_4$/$mm^3$ | Mai 90 | 396 | 780 |
|  |  | 22% | 29% |
| 2. % Lymphocytes $T_4$ (par rapport à l'ensemble des cellules mononuclées) | Avril 91 | 488 | 1297 |
|  |  | 21% | 37% |

(suite)

| | | K280 | G210 |
|---|---|---|---|
| Test de réaction intradermique | Mai 90 | + | - |
| | | - | - |
| 1. candidine | | ++ | - |
| 2. tuberculine | Avril 91 | + | ++ |
| Test CMI | Mai 90 | ND | ND |
| 1. candidine | Avril 91 | + | + |
| 2. tuberculine | | + | + |

Ces résultats révèlent une augmentation sensible de la population des lymphocytes $T_4$ entre Mai 90 et Avril 91 ainsi qu'une amélioration notable de la capacité du système immunitaire des patients à réagir à l'encontre d'un antigène.

## <u>EXEMPLE 8</u>

**Mise en évidence de l'activité immunosuppressive de peptides.**

Des cellules T issues de sang frais humain ont été mises en présence de macrophages présentant un antigène (SEB, candidine etc...) et en présence ou en l'absence des peptides à une concentration de 100 à 500 µg/ml.
Les cellules sont resuspendues dans du milieu classique de culture pour lymphocytes.
Après quatre jours on mesure l'activation des cellules par l'incorporation de thymidine marquée.
Alors que les cellules stimulées sans peptides ont un taux d'activation élevé, les cellules incubées avec un peptide selon l'invention présentent une baisse d'activation selon une courbe dose-effet pour les peptides IS N° 5, 6, 7 et 10.
Plus la quantité de peptides ajoutée est grande, plus on observe un abaissement de l'activité mesurée.

## <u>EXEMPLE 9</u>

**Effet des peptides immunogènes selon l'invention**

Quatre lots de cinq souris ont été choisis pour recevoir respectivement les peptides IS N°1, 2, 6, 10.
Les peptides préparés comme décrit à l'exemple 2 ont été injectés aux jours 0, 30, 60 en quantité de 15 µg par injection. Au jour 70, les souris ont été sacrifiées, leur cellules T ont été isolées (rate et ganglions) et leur sérum conservé.
Les tests de CMI, ELISA, Western blot on été réalisés :
En CMI, des cellules tueuses se sont manifestées in vitro après stimulation par le peptide immunisant mais pas par les autres peptides (mesure par test de relargage ou chrome 21).
En Elisa (plaques cotées avec les différents peptides) les sérums ont montré une positivité pour les plaques cotées par le peptide immunisant, et ce, à des dilutions très fortes (de l'ordre de 1 pour 750).
En Western Blot (kit de détection HIV-1 Abott), les bandes gp160 et gp120 ont montré un signal pour les peptides IS N° 1, 6, 10, et gp160 et gp41 pour le peptide IS N° 2.

## <u>EXEMPLE 10</u>

**Immunogénicité des virus déplétés selon l'invention.**

Des virus HIV-1 déplétés au formol ont été préparés comme décrit à l'exemple 5A. Une quantité de 1 mg/ml est associée à du phosphate de calcium comme décrit aux exemples 3 et 9.
20 µl de la suspension ainsi préparée sont injectées par voie intramusculaire à cinq souris au jours J 0, +30, +60. Au jour + 70 le sérum et les cellules T (rate) des souris sont prélevés.
En Elisa (kit de détection Abott), on trouve une réponse positive même à des dilutions élevées du sérum.
En Western blot (kit de détection Abott), on retrouve toutes les bandes classiquement connues (gp160, gp121 , p55, gp41).

En CMI, après stimulation par les peptides (le segment HGP30 de gag p17, peptide IS N° 4 de gp41) ou par du virus entier inactivé à la chaleur, ou encore par de la gp160 pure, on retrouve une activité cellulaire tueuse, que l'on n'observe pas chez des souris non immunisées.

## EXEMPLE 11

**Perte du pouvoir infectieux de virus déplétés selon l'invention.**

1 mg de virus HIV-1 déplété comme décrit à l'exemple 5A a été resuspendu dans 1 ml de RPMI 1640, FCS 10% et le tout ajouté à un culot de 5 millions de cellules sensibles à l'infection par le HIV-1 (cellules H9 et MT4).

Après deux heures d'incubation à 37°C, les cellules sont lavées deux fois dans du RPMI et remises en culture dans des conditions standard. L'ADN de ces cellules a été prélevé à 3 jours et à 8 jours, et on réalise une amplification par PCR (Polymerase Chain Reaction ou amplification génique) avec plusieurs amorces dans gag (SK17, SK18), pol, env. Ces tests ( capables de repérer trois copies d'ADN intégré) réalisés plusieurs fois se sont toujours montrés négatifs.

De la même façon, un test de PCR opéré après une étape de transcription inverse faite directement sur 1 mg de virus déplété donne une absence de signal témoignant d'une absence de génome viral dans le virus déplété (traité par l'hydroxyde de potassium à 0,3 N, incubé deux heures à 37°C et neutralisé par l'acide chlorhydrique, mais non formolé).

**Identificateur de séquence N° 1a et 1b**

1a. Peptide ayant une séquence identique à celle de la région de la sous-unité P7 de la protéine gag du virus HIV-1 sous couche HXB2, allant de l'acide aminé en position 377 à l'acide aminé en position 432*.

```
Ile-Ala-Arg-Asn-Cys-Arg-Ala-Pro-Arg-Lys-Lys-Gly.
```

1b.

```
Phe-Leu-Leu-Ala-Val-Phe-Cys-Ile-Ala-Arg-Asn-Cys-Arg-Ala-
Pro-Arg-Lys-Lys-Gly.
```

**Identificateur de séquence N° 2a et 2b.**

2a. Peptide ayant une séquence identique à celle de la région de la sous-unité gp41 de la protéine env du virus HIV-1 sous-souche HXB2, allant de l'acide aminé en position 735 à l'acide aminé en position 753*.

```
Asp-Arg-Pro-Glu-Gly-Ile-Glu-Glu-Glu-Gly-Gly-Glu-Arg-Asp-
Arg-Asp-Arg-Ser.
```

2b.

```
Phe-Leu-Leu-Ala-Val-Phe-Cys-Asp-Arg-Pro-Glu-Gly-Ile-Glu-
Glu-Glu-Gly-Gly-Glu-Arg-Asp-Arg-Asp-Arg-Ser.
```

* : Numérotation de Myers et al, retroviruses and AIDS (1990) Theorical Biology and Biophysics, Los Alamos Labs., Los Alamos NM 87745 USA, Ed. Myers et al ; cette numérotation varie légèrement selon la souche HIV choisie.

**Identificateur de séquence N° 3a et 3b.**

3a. Peptide ayant une séquence identique à celle de la région de la sous-unité gp41 de la protéine env du virus HIV-1 sous-souche HXB2, allant de l'acide aminé en position 834 à l'acide aminé en position 857*.

```
Glu-Gly-Thr-Asp-Arg-Val-Ile-Glu-Val-Val-Gln-Arg-Ala-Phe-
Arg-Ala-Ile-Leu-His-Ile-Pro-Arg-Arg-Ile-Arg-Gln.
```

3b.

```
Phe-Leu-Leu-Ala-Val-Phe-Cys-Glu-Gly-Thr-Asp-Arg-Val-Ile-
Glu-Val-Val-Gln-Arg-Ala-Phe-Arg-Ala-Ile-Leu-His-Ile-Pro-
Arg-Arg-Ile-Arg-Gln.
```

**Identificateur de séquence N° 4**

Peptide ayant une séquence identique à celle de la région de la sous-unité p15E de la protéine gag du virus HIV-1.

```
Leu-Gln-Asn-Arg-Arg-Gly-Leu-Asp-Leu-Leu.
```

**Identificateur de séquence N° 5**

Peptide ayant une séquence identique à celle de la région de la sous-unité gp41 de la protéine env du virus HIV-1 sous-souche HXB2, allant de l'acide aminé en position 560 à l'acide aminé en position 600*.

```
Cys-Gln-His-Leu-Leu-Gln-Leu-Thr-Val-Trp-Gly-Ile-Lys-Glu
ou Gln-Leu-Glu ou Gln-Ala-Arg-Ile-Leu-Ala-Val-Glu-Arg-
Tyr-Leu-Lys-Asp-Gln.
```

**Identificateur de séquence N° 6**

Le peptide SLWDQ issu de la protéine env du HIV-1 est totalement identique (résidus 110 à 114) à un segment du CD4 (résidus 60 à 64).

```
His-Glu-Asp-Ile-Ile-Ser-Leu-Trp-Asp-Gln-Ser-Leu-Lys.
```

* : Numérotation de Myers et al, retroviruses and AIDS (1990) Theorical Biology and Biophysics, Los Alamos Labs., Los Alamos NM 87745 USA, Ed. Myers et al ; cette numérotation varie légèrement selon la souche HIV choisie.

**Identificateur de séquence N° 7**

Le peptide CTASQK de la molécule CD4 (résidus 16-21) partage une forte homologie avec la protéine env du HIV-1 (CSATEK, résidus 28 à 33).


    Cys-Ser-Ala-Thr-Glu-Lys-Leu-Trp-Val-Thr-Val-Tyr-Tyr.


**Identificateur de séquence N° 8**

Le peptide EPTAPP est commun entre le CD5 et la protéine gag du HIV-1, et unique à ces deux molécules parmi toutes les protéines connues. Il correspond au résidus 454-459 de la protéine gag du HIV-1 et aux résidus 153-158 du récepteur CD5.


    Asn-Phe-Leu-Gln-Ser-Arg-Pro-Glu-Pro-Thr-Ala-Pro-Pro-Glu-
    Glu.


**Identificateur de séquence N° 9**

Le peptide TTLFCA (résidus 20 à 25) de la protéine env du HIV-1 a une très forte homologie avec le peptide TTLFCL du TNF-$\alpha$ (résidus 45 à 50).


    Ala-Gly-Ala-Thr-Thr-Leu-Phe-Cys-Leu-Leu-His.


**Identificateur de séquence N° 10**

Le peptide LLLNGSLA de la protéine env du HIV-1 (résidus 259-267) possède une similitude importante avec les molécules immunorégulatrices suivantes : HLA de classe I (résidus 11-18), chaîne $\beta$ du récepteur des cellules T (résidus 14 à 21), et G-CSF (résidus 17 à 24).


    Gln-Leu-Leu-Leu-Asn-Gly-Ser-Leu-Ala-Glu-Glu-Glu.


**Identificateur de séquence N° 11**

Le peptide QLTVW est commun à la protéine env du HIV-1 (résidus 566-570) et à la chaîne a du récepteur des cellules T (résidus 130-134).


    Gln-His-Leu-Leu-Gln-Leu-Thr-Val-Trp-Gly-Ile-Lys.


**Identificateur de séquence N° 12**

Le peptide GIRPVV de la protéine env du HIV-1 (résidus 250-256) a une très forte homologie avec le peptide GIRKVV de la chaîne a du précurseur de la protéine d'adhésion leucocytaire (résidus 230-236).


    His-Gly-Ile-Arg-Pro-Val-Val-Ser-Thr-Gln.

**Revendications**

1. Utilisation d'une cytokine immunogène chez l'homme, différente de sa forme native et inactivée, c'est à dire ayant perdu in vivo l'activité biologique caractéristique de ladite cytokine, par traitement chimique, physique ou immunologique, ou d'une cytokine inactive in vivo chez l'homme, c'est à dire une cytokine d'une espèce non humaine qui n'a pas in vivo chez l'homme l'activité biologique caractéristique de ladite cytokine, ou d'un analogue ou d'un fragment immunogène de ladite cytokine inactivé, c'est à dire ayant perdu in vivo l'activité biologique caractéristique de ladite cytokine, par traitement chimique, physique ou immunologique, ou d'un analogue ou d'un fragment inactif in vivo de ladite cytokine, c'est à dire un fragment d'une cytokine d'une espèce donnée mais dépourvue in vivo chez l'homme de l'activité biologique caractéristique de ladite cytokine, pour la préparation d'un agent immunisant chez l'homme.

2. Utilisation selon la revendication 1, caractérisée en ce que la cytokine, analogue ou fragment est d'origine humaine.

3. Utilisation selon la revendication 1 ou 2 d'une cytokine inactive ou inactivée qui est sélectionnée parmi un interféron-gamma, une interleukine-1, une interleukine-2, une interleukine-4, une interleukine-5, une interleukine-6, un tumor necrosis factor et un transforming growth factor-β ou d'un analogue ou fragment inactif ou inactivé desdites cytokines.

4. Utilisation selon la revendication 1 ou 2 d'une cytokine inactive ou inactivée qui est sélectionnée parmi un interféron-α, un interféron-β et un transforming growth factor-β, ou d'un analogue ou fragment inactif ou inactivé desdites cytokines.

5. Utilisation selon la revendication 4 d'une cytokine inactive ou inactivée qui est l'interféron-α ou d'un analogue ou fragment inactif ou inactivé de ladite cytokine.

6. Un procédé de préparation d'une cytokine, analogue ou fragment sous forme immunogène chez l'homme et inactivée tel que défini à la revendication 1, qui comprend l'acte d'inactiver une cytokine, analogue ou fragment en soumettant ladite cytokine, analogue ou fragment à un traitement chimique.

7. Un procédé selon la revendication 6, qui comprend l'acte d'inactiver une cytokine, analogue ou fragment actif en le soumettant à un traitement par un aldéhyde ou par la β-propiolactone.

8. Un procédé selon la revendication 6 ou 7, qui comprend l'acte d'inactiver une cytokine, analogue ou fragment actif en soumettant une cytokine, analogue ou fragment actif à la concentration de 10 μg à 100 μg/ml à un traitement par le formaldéhyde à une concentration finale de 10 mM à 100 mM, à une température de 10 à 60°C, pendant 2 à 15 jours.

9. Un procédé selon la revendication 8, dans lequel on inactive une cytokine active.

10. Une cytokine, analogue ou fragment sous forme inactivée et immunogène in vivo, susceptible d'être obtenue par le procédé tel que défini à la revendication 8 ou 9.

11. Une cytokine, analogue ou fragment selon la revendication 10, d'origine humaine.

12. Une cytokine, analogue ou fragment selon l'une des revendications 10 et 11, qui est sélectionnée parmi ceux obtenus à partir d'un interféron-gamma, une interleukine-1, une interleukine-2, une interleukine-4, une interleukine-5, une interleukine-6, un TNF et un transforming growth factor-β ou un de leurs fragments.

13. Une cytokine, analogue ou fragment selon l'une des revendications 10 et 11, qui est sélectionnée parmi ceux obtenus à partir d'un interféron-α, un interféron-β et un tumor growth factor-β.

14. Une cytokine, analogue ou fragment selon la revendication 13, qui est obtenue à partir de l'interféron-α.

15. Une composition pharmaceutique qui comprend, à titre d'agent thérapeutique, une cytokine, analogue ou fragment selon l'une des revendications 10 à 14.

16. Une composition pharmaceutique selon la revendication 15, qui comprend en outre un agent vaccinal conventionnel d'origine microbienne.

**17.** Utilisation selon l'une des revendications 1 à 5, caractérisée en ce que l'agent immunisant est destiné au traitement d'une infection à HIV au stade ARC ou SIDA ou d'une manifestation clinique associée à une infection à HIV.

**18.** Utilisation selon l'une des revendications 1 à 5, caractérisée en ce que l'agent immunisant est destiné au traitement d'un cancer, d'une affection neurologique, d'une affection auto-immune ou d'une allergie.

**19.** Utilisation selon l'une des revendications 1 à 5, caractérisée en ce que le traitement est un traitement chimique.

**20.** Utilisation selon l'une des revendications 1 à 5, caractérisée en ce que le traitement est un traitement chimique par un aldéhyde ou par la β-propiolactone.

**21.** Utilisation selon l'une des revendications 1 à 5, caractérisée en ce que l'on utilise une cytokine telle que définie à l'une des revendications 10 à 14.

**22.** Une cytokine immunogène chez l'homme, différente de sa forme native et inactivée, c'est à dire ayant perdu in vivo l'activité biologique caractéristique de ladite cytokine, par traitement chimique, physique ou immunologique, ou d'une cytokine inactive chez l'homme, c'est à dire une cytokine d'une espèce non humaine qui n'a pas in vivo chez l'homme l'activité biologique caractéristique de ladite cytokine, ou d'un analogue ou d'un fragment immunogène de ladite cytokine inactivé, c'est à dire ayant perdu in vivo l'activité biologique caractéristique de ladite cytokine, par traitement chimique, physique ou immunologique, ou d'un analogue ou d'un fragment inactif de ladite cytokine, c'est à dire un fragment d'une cytokine d'une espèce donnée mais dépourvue in vivo chez l'homme de l'activité biologique caractéristique de ladite cytokine, pour son utilisation dans une méthode de traitement thérapeutique chez l'homme.

**23.** Une cytokine, analogue ou fragment selon la revendication 22, telle que définie à l'une des revendications 2 à 5, pour son utilisation dans une méthode de traitement thérapeutique chez l'homme.

**24.** Une cytokine, analogue ou fragment selon la revendication 22, telle que définie à l'une des revendications 10 à 14, pour son utilisation dans une méthode de traitement thérapeutique chez l'homme.

**25.** Un vaccin caractérisé en ce qu'il comprend, à titre d'agent immunisant, une cytokine immunogène chez l'homme, différente de sa forme native et inactivée, c'est à dire ayant perdu in vivo l'activité biologique caractéristique de ladite cytokine, par traitement chimique, physique ou immunologique, ou d'une cytokine inactive chez l'homme, c'est à dire une cytokine d'une espèce non humaine qui n'a pas in vivo chez l'homme l'activité biologique caractéristique de ladite cytokine, ou d'un analogue ou d'un fragment immunogène de ladite cytokine inactivé, c'est à dire ayant perdu l'activité biologique caractéristique de ladite cytokine, par traitement chimique, physique ou immunologique, ou d'un analogue ou d'un fragment inactif de ladite cytokine, c'est à dire un fragment d'une cytokine d'une espèce donnée mais dépourvue in vivo chez l'homme de l'activité biologique caractéristique de ladite cytokine.

**26.** Un vaccin selon la revendication 26, caractérisé en ce qu'il comprend à titre d'agent immunisant, une cytokine, analogue ou fragment tel que défini à l'une des revendications 2 à 5 et 10 à 14.

**27.** Un vaccin selon la revendication 25 ou 26, caractérisé en ce qu'il comprend en outre un adjuvant.

**28.** Un kit thérapeutique renfermant à titre d'agent thérapeutique, une cytokine, analogue ou fragment tel que défini à l'une des revendications 1 à 5 qui est un tumor necrosis factor, un transforming growth factor-β, un interféron-α ou un interféron-β.

- une composition pharmaceutique ,
- une composition vaccinale conventionnelle,
- des instructions pour l'administration concomitante ou consécutive des deux compositions ci-dessus.

**Claims**

1. Use of an immunogenic cytokine in man, different from its native and inactive form, that is, having lost in vivo the biological activity characteristic of said cytokine, by chemical, physical or immunological treatment, or of an inactive in vivo cytokine in man, that is, a cytokine of a non-human species which does not have in vivo in man the biological activity characteristic of said cytokine, or of an analogue or of an immunogenic fragment of said inactivated cytokine, that is, having lost in vivo the biological activity characteristic of said cytokine, by chemical, physical or immunological treatment, or of an analogue or of an inactive fragment of said cytokine in vivo, that is, a fragment of a cytokine of a given species but lacking in vivo in man the biological activity characteristic of said cytokine, for the preparation of an immunising agent in man.

2. Use, according to claim 1, characterized in that the cytokine, analogue or fragment, is of human origin.

3. Use, according to claim 1 or 2, of an inactive or inactivated cytokine which is selected from a gamma interferon, an interleukin-1, an interleukin-2, an interleukin-4, and interleukin-5, an interleukin-6, a tumour necrosis factor and a transforming growth factor-β or an analogue or inactive or inactivated fragment of said cytokines.

4. Use. according to claim 1 or 2 of an inactive or inactivated cytokine which is selected from an interferon-α, an interferon-β and a transforming growth factor-β, or an analogue or inactive or inactivated fragment of said cytokines.

5. Use, according to claim 4 of an inactive or inactivated cytokine which is interferon-α or an analogue or inactive or inactivated fragment of said cytokine.

6. A preparation process for a cytokine, analogue or fragment, in immunogenic form in man and inactivated as defined in claim 1, which comprises the action of inactivating a cytokine, analogue or fragment by subjecting said cytokine, analogue or fragment to a chemical treatment.

7. A process according to claim 6 which comprises the action of inactivating a cytokine, analogue or active fragment by subjecting it to a treatment by an aldehyde or by β-propiolactone.

8. A process according to claim 6 or 7 which comprises the act of inactivating a cytokine, analogue or active fragment by subjecting a cytokine, analogue or inactive fragment at a concentration of 10 μg to 100 μg/ml to a treatment by formaldehyde at a final concentration of 10mM to 100mM, at a temperature of 10 to 60°C for 2 to 15 days.

9. A process according to claim 8 in which an active cytokine is inactivated.

10. A cytokine, analogue or fragment in inactivated and immunogenic form, susceptible to being obtained by the process as defined in claim 8 or 9.

11. A cytokine, analogue or fragment according to claim 10, of human origin.

12. A cytokine, analogue or fragment according to one of claims 10 to 11, which is selected from those obtained from an interferon-gamma, an interleukin-1, an interleukin-2, an interleukin-4, an interleukin-5, an interleukin-6, a TNF and a transforming growth factor-β or one of their fragments.

13. A cytokine, analogue or fragment according to one of claims 10 and 11, which is selected from those obtained from an interferon-α, an interferon-β and a tumor growth factor-β.

14. A cytokine, analogue or fragment according to claim 13, which is obtained from interferon-α.

15. A pharmaceutical composition which contains a cytokine, analogue or fragment according to one of claims 10 to 14 as therapeutic agent.

16. A pharmaceutical composition according to claim 15 which contains, in addition, a conventional vaccinal agent of microbial origin.

17. Use, according to one of claims 1 to 5, characterized in that the immunizing agent is intended to treat an HIV infection at the ARC or AIDS stage or a clinical manifestation associated with a HIV infection.

18. Use, according to one of claims 1 to 5, characterized in that the immunising agent is intended to treat a cancer, a neurological affection, an auto-immune affection or an allergy.

19. Use, according to one of claims 1 to 5, characterized in that the treatment is a chemical treatment.

20. Use, according to one of claims 1 to 5, characterized in that the treatment is a chemical treatment by an aldehyde or by β-propiolactone.

21. Use, according to one of claims 1 to 5, characterized in that a cytokine as defined in one of claims 10 to 14 is used.

22. An immunogenic cytokine in man, different from its native form and inactivated, that is, having lost in vivo the biological activity characteristic of said cytokine, by chemical, physical or immunological treatment, or an inactive cytokine in man, that is a cytokine of a non-human species which does not have in vivo in man the biological activity characteristic of said cytokine, or of an analogue or of immunogenic fragment of said inactivated cytokine, that is, having lost in vivo the biological activity characteristic of said cytokine by chemical, physical or immunological treatment, or an analogue or inactive fragment of said cytokine, that is, a fragment of a cytokine of a given species but lacking in vivo in man the biological activity characteristic of said cytokine, for its use in a therapeutic method of treatment in man.

23. A cytokine, analogue or fragment according to claim 22, as defined in one of claims 2 to 5, for its use in a therapeutic method of treatment in man.

24. A cytokine, analogue or fragment according to claim 22, as defined in one of claims 10 to 14, for its use in a therapeutic method of treatment in man.

25. A vaccine characterized in that it comprises, as an immunising agent, an immunogenic cytokine in man, different from its native form and inactivated, that is, having lost in vivo the biological activity characteristic of said cytokine, by chemical, physical or immunological treatment, or an inactive cytokine in man, that is a cytokine of a non-human species which does not have in vivo in man the biological activity characteristic of said inactive cytokine or an analogue or an immunogenic fragment of said inactivated cytokine, that is, having lost the biological activity characteristic of said cytokine, by chemical, physical or immunological treatment, or of an analogue or inactive fragment of said cytokine, that is a fragment of cytokine of a given species but lacking in vivo in man the biological activity characteristic of said cytokine.

26. A vaccine according to claim 26, characterized in that it contains as an immunizing agent, a cytokine, analogue or fragment as defined in one of claims 2 to 5 and 10 to 14.

27. A vaccine according to claim 25 or 26, characterized in that it contains, in addition, an adjuvant.

28. A therapeutic kit containing, as a therapeutic agent, a cytokine, analogue or fragment as defined in one of claims 1 to 5, which is a tumor necrosis factor, a transforming growth factor-β, an interferon-α or an interferon-β.

   - a pharmaceutical composition,
   - a conventional vaccinal composition,
   - instructions for the concomitant or consecutive administration of the two compositions above.


**Patentansprüche**

1. Verwendung eines beim Menschen immunogenen Cytokins, das verschieden von seiner natürlichen Form und inaktiviert vorliegt, d.h. das durch chemische, physikalische oder immunologische Behandlung die charakteristische biologische in vivo Aktivität des Cytokins verloren hat, oder eines beim Menschen in vivo inaktiven Cytokins, d.h. eines Cytokins einer nichthumanen Spezies, das die charakteristische biologische in vivo Aktivität des Cytokins beim Menschen nicht besitzt, oder eines immunogenen Analogons oder Fragments des inaktivierten Cytokins, d. h. das durch chemische, physikalische oder immunologische Behandlung die charakteristische biologische in vivo Aktivität des Cytokins verloren hat, oder eines in vivo inaktiven Analogons oder Fragments des Cytokins, d.h. ein Fragment eines Cytokins einer vorgegebenen Spezies, aber ohne die charakteristische biologische in vivo Aktivität des Cytokins beim Menschen, für die Herstellung eines Immunisierungsmittels beim Menschen.

2. Verwendung nach Anspruch 1, dadurch gekennzeichnet, daß das Cytokin, Analogon oder Fragment menschlichen Ursprungs ist.

3. Verwendung eines inaktiven oder inaktivierten Cytokins nach Anspruch 1 oder 2, das ausgewählt ist aus einem gamma-Interferon, einem 1-Interleukin, einem 2-Interleukin, einem 4-Interleukin, einem 5-Interleukin, einem 6-Interleukin, einem Tumor-Nekrose-Faktor und einem β-Transforming-Growth-Faktor oder einem inaktiven oder inaktivierten Analogon oder Fragment der Cytokine.

4. Verwendung eines inaktiven oder inaktivierten Cytokins nach Anspruch 1 oder 2, das ausgewählt ist aus einem α-Interferon, einem β-Interferon und einem β-Transforming-Growth-Faktor oder einem inaktiven oder inaktivierten Analogon oder Fragment der Cytokine.

5. Verwendung eines inaktiven oder inaktivierten Cytokins nach Anspruch 4, das α-Interferon oder ein inaktives oder inaktiviertes Analogon oder Fragment des Cytokins ist.

6. Verfahren zur Herstellung eines Cytokins, Analogons oder Fragments in beim Menschen immunogener Form und inaktiviert, wie in Anspruch 1 definiert, das das Inaktivieren eines Cytokins, Analogons oder Fragments umfaßt, indem das Cytokin, Analogon oder Fragment einer chemischen Behandlung unterworfen wird.

7. Verfahren nach Anspruch 6, das das Inaktivieren eines aktiven Cytokins, Analogons oder Fragments umfaßt, indem es einer Behandlung mit einem Aldehyd oder mit β-Propiolacton unterzogen wird.

8. Verfahren nach Anspruch 6 oder 7, das das Inaktivieren eines aktiven Cytokins, Analogons oder Fragments, umfaßt, indem das aktive Cytokin, Analogon oder Fragment mit einer Konzentration von 10 µg bis 100 µg/ml einer Behandlung mit Formaldehyd einer Endkonzentration von 10 mM bis 100 mM bei einer Temperatur von 10 bis 60°C für 2 bis 15 Tage unterzogen wird.

9. Verfahren nach Anspruch 8, worin man ein aktives Cytokin inaktiviert.

10. Cytokin, Analogon oder Fragment in inaktivierter und in vivo immunogener Form, erhältlich nach dem Verfahren, definiert in den Ansprüchen 8 oder 9.

11. Cytokin, Analogon oder Fragment nach Anspruch 10, menschlichen Ursprungs.

12. Cytokin, Analogon oder Fragment nach einem der Ansprüche 10 oder 11, das ausgewählt ist unter denen, erhalten aus einem garnma-Interferon, einem 1-Interleukin, einem 2-Interleukin, einem 4-Interleukin, einem 5-Interleukin, einem 6-Interleukin, einem TNF und einem β-Transforming-Growth-Faktor oder einem ihrer Fragmente.

13. Cytokin, Analogon oder Fragment nach einem der Ansprüche 10 oder 11, das ausgewählt ist unter denen. erhalten aus einem α-Interferon, einem β-Interferon und einem β-Tumor-Wachstums-Faktor.

14. Cytokin, Analogon oder Fragment nach Anspruch 13, das ausgehend von α-Interferon erhalten wird.

15. Pharmazeutische Zusammensetzung, die als therapeutisches Mittel ein Cytokin, Analogon oder Fragment nach einem der Ansprüche 10 bis 14 umfaßt.

16. Pharmazeutische Zusammensetzung nach Anspruch 15, die zudem ein Vakzin herkömmlichen mikrobiellen Ursprungs umfaßt.

17. Verwendung nach einem der Ansprüche 1 bis 5, dadurch gekennzeichnet, daß das Immunisierungsmittel zur Behandlung einer HIV-Infektion im ARC- oder AIDS-Stadium oder bei mit einer HIV-Infektion verbundenen klinischen Ausprägungen bestimmt ist.

18. Verwendung nach einem der Ansprüche 1 bis 5, dadurch gekennzeichnet, daß das Immunisierungsmittel zur Behandlung von Krebs, einer neurologischen Erkrankung, einer Autoimmunerkrankung oder einer Allergie bestimmt ist.

19. Verwendung nach einem der Ansprüche 1 bis 5, dadurch gekennzeichnet, daß die Behandlung eine chemische Behandlung ist.

20. Verwendung nach einem der Ansprüche 1 bis 5, dadurch gekennzeichnet, daß die Behandlung eine chemische Behandlung mit einem Aldehyd oder β-Propiolacton ist.

21. Verwendung nach einem der Anspüche 1 bis 5, dadurch gekennzeichnet, daß man ein Cytokin, wie es in einem der Ansprüche 10 bis 14 definiert ist, einsetzt.

22. Beim Menschen immunogenes Cytokin, das verschieden von seiner natürlichen Form und inaktiviert vorliegt, d. h. das durch chemische, physikalische oder immunologische Behandlung die charakteristische biologische in vivo Aktivität des Cytokins verloren hat, oder ein beim Menschen inaktives Cytokin, d.h. ein Cytokin einer nichthumanen Spezies, das die charakteristische biologische in vivo Aktivität des Cytokins beim Menschen nicht besitzt, oder ein immunogenes Analogon oder Fragment des inaktivierten Cytokins, d.h. das durch chemische, physikalische oder immunologische Behandlung die charakteristische biologische in vivo Aktivität des Cytokins verloren hat, oder ein inaktives Analogon oder Fragment des Cytokins, d.h. ein Fragment eines Cytokins einer vorgegebenen Spezies, aber ohne die charakteristische biologische in vivo Aktivität des Cytokins beim Menschen, zur Verwendung in einem therapeutischen Behandlungsverfahren beim Menschen.

23. Cytokin. Analogon oder Fragment nach Anspruch 22, wie es nach einem der Ansprüche 2 bis 5 definiert ist, zur Verwendung in einem therapeutischen Behandlungsverfahren beim Menschen.

24. Cytokin, Analogon oder Fragment nach Anspruch 22, wie es in einem der Ansprüche 10 bis 14 definiert ist, zur Verwendung in einem therapeutischen Behandlungsverfahren beim Menschen.

25. Vakzin, dadurch gekennzeichnet, daß es als Immunisierungsmittel ein beim Menschen immunogenes Cytokin umfaßt, das verschieden von seiner natürlichen und inaktivierten Form vorliegt, d.h. das durch chemische, physikalische oder immunologische Behandlung die charakteristische biologische in vivo Aktivität des Cytokins verloren hat, oder ein beim Menschen inaktives Cytokin, d.h. ein Cytokin einer nichthumanen Spezies, das die charakteristische biologische in vivo Aktivität des Cytokins beim Menschen nicht besitzt, oder ein immunogenes Analogon oder Fragment des inaktivierten Cytokins, d.h. das durch chemische, physikalische oder immunologische Behandlung die charakteristische biologische in vivo Aktivität des Cytokins verloren hat, oder ein inaktives Analogon oder Fragment des Cytokins, d.h. ein Fragment eines Cytokins einer vorgegebenen Spezies, aber ohne die charakteristische biologische in vivo Aktivität des Cytokins beim Menschen.

26. Vakzin nach Anspruch 25, dadurch gekennzeichnet, daß es als Immunisierungsmittel ein Cytokin, Analogon oder Fragment, umfaßt, wie es in einem der Ansprüche 2 bis 5 und 10 bis 14 definiert ist.

27. Vakzin nach Anspruch 25 oder 26, dadurch gekennzeichnet, daß es zudem einen Hilfsstoff umfaßt.

28. Therapeutisches Kit, enthaltend als therapeutisches Mittel ein Cytokin, Analogon oder Fragment, wie es in einem der Ansprüche 1 bis 5 definiert ist, das ein Tumor-Nekrose-Faktor, ein β-Transforming-Growth-Factor, ein α-Interferon oder ein β-Interferon ist,

- eine pharmazeutische Zusammensetzung,
- eine herkömmliche Vakzinzusammensetzung,
- Anweisungen zur gleichzeitigen oder aufeinanderfolgenden Verabreichung der beiden obigen Zusammensetzungen.